# EUROPEAN PATENT APPLICATION

(11) **EP 2 275 115 A2**
(43) Date of publication of application: **19.01.2011**
(21) Application number: 10182821.8
(22) Date of filing: 05.07.2004
(51) Int. Cl.: A61K 38/00, A61K 48/00, A61K 39/395

(54) **Inhibition of stress-induced ligand-dependent EGFR activation**

(30) Priority: 04.07.2003 EP 03015209
(62) Divisional of application: 04740660.8
(71) Applicant: Max-Planck-Gesellschaft zur Förderung der Wissenschaften E.V., 80539 München (DE)
(72) Inventor: Ullrich, Axel, 80331 München (DE); Fischer, Oliver, 12099 Berlin (DE)
(74) Representative: Weiss, Wolfgang

(57) **Abstract**

The present invention relates to the inhibition of stress-induced receptor tyrosine kinase activity by inhibiting a ligand of said receptor tyrosine kinase, particularly an extracellular ligand.
Further, the present invention relates to the use of such inhibitors for the manufacture of a medicament for the prevention or treatment of an at least partially therapy-resistant hyper proliferative disorder.

## Description

The present invention relates to the inhibition of stress-induced receptor tyrosine kinase activity by inhibiting a ligand of said receptor tyrosine kinase, particularly an extracellular ligand.

Exposure of mammalian cells to environmental stress such as hyperosmolarity and oxidative agents, ionizing radiation or UV-light activates a variety of signal transduction cascades. While reactive oxygen species (ROS) have been implicated as second messengers, oxidative stress due to their uncontrolled production and exposure to oxidants has been related to cellular damage and pathophysiological disorders such as cancer (Finkel, 1998; Kamata and Hirata, 1999). Moreover, mammalian cells have to adapt to changes in the extracellular environment including increasing osmolarity, which results in cell shrinkage and increased synthesis of small molecules to equalize the intra- and extracellular conditions (de Nadal et al., 2002).

Osmotic or oxidative stress activate a variety of receptor tyrosine kinases, the most prominent being the epidermal growth factor receptor (EGFR) (King et al., 1989; Knebel et al., 1996; Rao, 1996; Rosette and Karin, 1996). The EGFR controls a plethora of important biological responses including cell proliferation, differentiation, migration or antiapoptotic signals, and has therefore frequently been implicated in diverse human disorders (Prenzel et al., 2001). Ligand-dependent and -independent receptor activation mechanisms have been described for the EGFR. Ligand-mediated receptor activation occurs by binding of an EGF-like ligand, such as EGF, HB-EGF, amphiregulin or TGF-α to receptor ectodomains leading to dimerization of two ligand-receptor heterodimers, subsequent activation of the intrinsic kinase activity and autophosphorylation (Schlessinger, 2002). Ligand-independent receptor activation has been proposed to occur via inactivation of phosphatases involving oxidation of a critical cysteine residue within their catalytic pocket (Knebel et al., 1996). Hence, the equilibrium of receptor phosphorylation is shifted from the non-phosphorylated to the phosphorylated state. Another mechanism for ligand-independent receptor activation has been suggested to involve non-specific clustering and internalization of the EGFR (Rosette and Karin, 1996). Furthermore, cytoplasmic non-receptor tyrosine kinases such as c-Src have been shown to phosphorylate the EGFR (Biscardi et al., 1999; Tice et al., 1999).

Tyrosine phosphorylation of the EGFR can also be induced by G protein-coupled receptor (GPCR) stimulation, a process that has been termed EGFR transactivation (Daub et al., 1996). The mechanism has originally been attributed to an exclusively ligand-independent process. In many cell systems however, EGFR transactivation occurs via metalloprotease-mediated shedding of transmembrane EGF-like ligands that have to be processed to become active (Prenzel et al., 1999). Very recently members of the ADAM family of metalloproteases were identified as the sheddases required for GPCR-induced proHB-EGF and pro-AR processing (Gschwind et al., 2003; Yan et al., 2002). Above all, ADAM17, also named TNF-α converting enzyme (TACE), but also ADAM10 and ADAM12 have been shown to be involved (Asakura et al., 2002; Gschwind et al., 2003; Yan et al., 2002). Aberrant signalling processes involving ligand-dependent EGFR transactivation have been related to different human disorders, such as head and neck squamous cell carcinoma, cardiac and gastrointestinal hypertrophy and cystic fibrosis (Asakura et al., 2002; Gschwind et al., 2002; Keates et al., 2001; Lemjabbar and Basbaum, 2002). Apart from EGFR transactivation ADAM9 has been shown to process proHB-EGF in response to TPA stimulation (Izumi et al., 1998), while also cleavage of proTGF-α, proHB-EGF and proamphiregulin by ADAM17 has been reported (Merlos-Suarez et al., 2001; Peschon et al., 1998; Sunnarborg et al., 2002) and implicated in tumourigenesis in the case of TGF-α (Borrell-Pages et al., 2003).

Besides RTK phosphorylation, environmental stress leads to the activation of mitogen-activated protein kinases (MAPKs) which are part of major intracellular signal transduction cascades coupling extracellular stimuli to the nucleus by activating transcription factors. Thus, MAPK pathways control important processes such as proliferation, migration, differentiation and stress responses (Chen et al., 2001b; Johnson and Lapadat, 2002). However, apart from activating transcription factors the MAPKs extracellular signal-regulated kinase-1/2 (ERK1/2) and p38 have been implicated in controlling processing of transmembrane proteins (Fan and Derynck, 1999) by phosphorylating the intracellular domain of ADAM17 (Diaz-Rodriguez et al., 2002; Fan et al., 2003). Intensive research has focused on mechanisms of MAPK activation by osmotic and oxidative stress (Kyriakis and Avruch, 2001) as the cell's fate is determined by cross-talk between these signalling pathways. Previous reports proposed the inactivation or downregulation of phosphatases in MAPK activation due to stress agents as well as the modification of scaffolding protein function, leading to association or dissociation of signalling complexes (Benhar et al., 2002; de Nadal et al., 2002). Moreover, small G-proteins have been demonstrated to play a role in the activation of MAPK by osmotic stress (de Nadal et al., 2002). The activation of MAPK by stress stimuli has severe consequences for the development and progression of human cancer as increasing evidence implicates particularly ROS, the stress-activated kinases p38 and JNK and stress signalling in the susceptibility of cancer cells to apoptosis and in proliferative responses. Thereby, stress signalling via the EGFR can affect cancer therapy, as recent studies indicate that anti-cancer drugs activate stress signalling cascades (reviewed in Benhar et al., 2002).

The mechanisms of RTK and MAPK activation in response to oxidative and osmotic stress have been intensively studied, but so far, these regulatory pathways were generally described as ligand-independent processes in human carcinoma cells. Recent advances in understanding the regulation of EGFR activation by ADAM metalloproteases prompted us to investigate the mechanisms of stress-induced EGFR and MAPK stimulation with respect to a potential involvement of EGF-like ligand processing.

Fischer et al. (Poster PS01-0916, Eur. J. Biochem.) describe that p38 and metalloproteases of the ADAM family control stress-induced ligand-dependent EGFR activation in downstream signalling in human carcinoma cells. Specific metalloproteases are, however, not identified.

Takenobu et al. (J. Biol. Chem. 278, (2003), 1725-1762) disclose stress- and inflammatory cytokine-induced ectodomain shedding of HB-EGF-like growth factors mediated by p38 MAPK. It was found that the metalloprotease ADAM9 is not required for stress-induced pro HB-EGF shedding in VeroH cells.

Herrlich et al. (FASEB J. 16 (2002), A56) and Tschumperlin et al. (FASEB J. 16 (2002), A1150) disclose stimulation of HER2/HER3 or EGFR activation respectivelyby heregulin shedding mediated by osmotic or mechanical stress.

In the present application it is now demonstrated that EGFR activation in response to osmotic or oxidative stress involves metalloprotease-mediated cleavage of proHB-EGF. The responsible metalloproteases comprise members of the ADAM family processing proHB-EGF, particularly ADAM9, ADAM10 and ADAM17. Furthermore, stress-induced ligand-dependent EGFR activation can be linked to the MAPKs ERK1/2 and JNK. We provide evidence that stress-activated EGFR phosphorylation depends on p38 activity, implicating p38 as an upstream activator of ADAM metalloproteases in the stress response of human carcinoma cells.

Further a combination treatment of tumour cells with a chemotherapeutic agent, e.g. with doxorubicin, which induces p38 activation, and blockade of HB-EGF effect strongly enhanced cell death when compared to doxorubicin treatment alone. This result suggests a role of this signalling mechanism for tumour cells to escape chemotherapy-induced cell death.

Thus, a method is provided which allows modulating of stress-induced activation of a receptor tyrosine kinase or an RTK-mediated signalling pathway in a cell, preferably in a mammalian cell, more preferably in a human cell, e.g. in a tumour cell, in particular in a hyperproliferative or apoptosis-resistant cell comprising inhibiting the activity of a ligand of said receptor tyrosine kinase.

A first aspect of the invention relates to the use of an inhibitor of a receptor tyrosine kinase ligand for the manufacture of a medicament for the prevention or treatment of an at least partially therapy-resistant hyperproliferative disorder.

A further aspect of the present invention relates to the use of an inhibitor of a receptor tyrosine kinase ligand for increasing the efficacy of therapies against hyperproliferative disorders.

A further aspect of the present invention relates to the use of an inhibitor of a receptor tyrosine kinase ligand for the manufacture of a medicament for increasing the sensitivity of hyperproliferative disorders against irradiation and/or medicament treatment.

Still a further aspect of the present invention relates to the use of an inhibitor of a receptor tyrosine kinase ligand for the manufacture of a medicament for the prevention or treatment of a disorder which is caused by or associated with stress-induced activation of a receptor tyrosine kinase.

Still a further aspect of the present invention is a pharmaceutical composition or kit comprising as active ingredients
a) an inhibitor of a receptor tyrosine kinase ligand which is an inhibitor of a metalloprotease capable of cleaving the receptor tyrosine kinase ligand or an inhibitor of regulatory steps upstream of the metalloprotease,
and
b) a further medicament for the treatment of hyperproliferative disorders.

The term "receptor tyrosine kinase" is well understood in the art and preferably relates to membrane-bound molecules having tyrosine kinase activity which are comprised of an extracellular domain, a transmembrane domain and an intracellular domain. Examples of suitable receptor tyrosine kinases are EGFR and other members of the EGFR family such as HER2, HER3 or HER4 as well as other receptor tyrosine kinases such as PDGFR, the vascular endothelial growth factor receptor KDR/FLK-1, the TRK receptor, FGFR-1 or IGF-1 receptor but also other types of growth factor receptors such as TNF receptor 1, TNF receptor 2, CD30 and IL6 receptor. Preferably, the receptor tyrosine kinase is EGFR.

The present invention comprises the inhibition of the activity of a receptor tyrosine kinase ligand. The inhibition is preferably a "specific" inhibition, wherein the activity of a specific receptor tyrosine kinase ligand is selectively inhibited, i.e. the activity of other receptor tyrosine kinase ligands is not significantly inhibited. By means of selective inhibition of specific receptor tyrosine kinase ligands a highly specific disruption of receptor tyrosine kinase activity may be achieved which is important for pharmaceutical applications in that the occurrence of undesired side effects may be reduced. It should be noted, however, that the method of the present invention also comprises a non-specific inhibition of receptor tyrosine kinase ligands.

Further, the invention preferably relates to an inhibition, wherein an inhibitor acts directly on the receptor tyrosine kinase ligand itself or on a metalloprotease capable of cleaving the receptor tyrosine kinase ligand, e.g. by binding. The invention, however, also encompasses an inhibition wherein the inhibitor does not directly act on the metalloprotease and/or the receptor tyrosine kinase ligand but to a precursor or metabolite thereof, particularly a precursor of the receptor tyrosine kinase ligand. Furthermore, the invention also relates to an inhibition of p38 for the modulation of stress-induced receptor tyrosine kinase activity.

The receptor tyrosine kinase ligand is preferably a molecule binding to the extracellular receptor tyrosine kinase domain. Examples of suitable receptor tyrosine kinase ligands are HB-EGF, EGF, amphiregulin, betacellulin, epiregulin, TGF-α, neuregulin or heregulin. More preferably, the receptor tyrosine kinase ligand is HB-EGF.

The inhibition of the activity of a receptor tyrosine kinase ligand preferably relates to an inhibition of the cleavage of a precursor, particularly a membrane-bound precursor of the ligand by a metalloprotease and/or the activation of a receptor tyrosine kinase, e.g. EGFR by the ligand. A metalloprotease inhibitor of the present invention is preferably capable of inhibiting the cleavage of the ligand precursor and its release. Examples of suitable metalloproteases are ADAM9, 10 and 17, particularly ADAM17 which are critical mediators of the cellular stress response. Alternatively, the inhibitor of the present invention may be capable of inhibiting the biological activity of the receptor tyrosine kinase ligand, particularly EGFR tyrosine phosphorylation, downstream mitogenic signalling events, e.g. activation of mitogen-activated protein kinases (MAPKs) such as ERK1/2 and/or JNK, cell proliferation and/or migration.

Inhibitors of a receptor tyrosine kinase ligand may be used for the prevention or treatment of disorders, particularly hyperproliferative disorders. Preferably the disorder is caused by or associated with stress-induced activation of a receptor tyrosine kinase. The stress is preferably an oxidative and/or osmotic stress. More preferably, the stress is a p38-mediated stress. The presence of such a type of disorder may be determined by measuring p38 expression, e.g. on mRNA level (cDNA array analysis, SAGE, Northern Blot, etc) and/or on the protein level (Western Blot analysis, immunofluoresence microscopy, in situ hybridization techniques, etc). The presence of such type of disorder may also be determined by examining the occurrence of activating mutations in genomic and/or mRNA molecules encoding p38. Further, elevated levels of p38 agonists in serum and/or disease-affected tissues may be determined. It should be pointed out that this type of disorder need not be associated with enhanced receptor tyrosine kinase expression.

For example, the disorder may be a hyperproliferative disorder such as a cancer, e.g. breast, stomach, prostate, bladder, ovarial, lung, liver, kidney or pancreas cancer, glioma, melanoma, leukemia, etc or another disorder such as a hyperproliferative skin disease, e.g. psoriasis or inflammatory diseases.

The activity of receptor tyrosine kinase ligands (preferably either directly or via metalloprotease inhibition) may be inhibited on the nucleic acid level, e.g. on the gene level or on the transcription level.

Inhibition on the gene level may comprise a partial or complete gene inactivation, i.e. by gene disruption. On the other hand, inhibition may occur on the transcript level, e.g. by application of anti-sense molecules, e.g. DNA molecules , RNA molecules or nucleic acid analogues, ribozymes, e.g. RNA molecules or nucleic acid analogues or small RNA molecules capable of RNA interference (RNAi), e.g. RNA molecules or nucleic acid analogues, directed against metalloprotease and/or ligand mRNA.

Further, the activity may be inhibited on the protein level, e.g. by application of compounds which result in a specific inhibition of a metalloprotease and/or ligand activity. The inhibition on the protein level may comprise, for example, the application of antibodies or antibody fragments directed against a metalloprotease such as ADAM9, 10 or 17 or a ligand or ligand precursor such as HB-EGF or PreHB-EGF. The antibodies may be polyclonal antibodies or monoclonal antibodies, recombinant antibodies, e.g. single chain antibodies or fragments of such antibodies which contain at least one antigen-binding site. e.g. proteolytic antibody fragments such as Fab, Fab' or F(ab')₂ fragments or recombinant antibody fragments such as scFv fragments. For therapeutic purposes, particularly for the treatment of humans, the application of chimeric antibodies, humanized antibodies or human antibodies is especially preferred.

Furthermore, proteinaceous or low-molecular weight inhibitors of metalloproteases and/or ligands may be used. Examples of such inhibitors are CRM197, batimastat, marimastat, heparin or blocking antibodies against the ligands. Further inhibitors may be identified by screening procedures as outlined in detail below.

For therapeutic purposes, the medicament is administered in the form of a pharmaceutical composition which additionally comprises pharmaceutically acceptable carriers, diluents and/or adjuvants.

Pharmaceutical compositions suitable for use in the present invention include compositions wherein the active ingredients are contained in an effective amount to achieve its intended purpose. A therapeutically effective dose refers to that amount of the compound that results in amelioration of symptoms or a prolongation of survival in a patient. Toxicity and therapeutic efficacy of such compounds can be determined by standard pharmaceutical procedures in cell cultures or experimental animals, e.g. for determining the LD50 (the dose lethal to 50% of the population) and the ED50 (the dose therapeutically effective in 50% of the population). For any compound used in the method of the invention, the therapeutically effective dose can be estimated initially from cell culture assays. For example, a dose can be formulated in animal models to achieve a circulating concentration range that includes the IC50 as determined in cell culture (i.e. the concentration of the test compound which achieves a half-maximal inhibition of the growth-factor receptor activity). Such information can be used to more accurately determine useful doses in humans. The dose ration between toxic and therapeutic effects is the therapeutic index and it can be expressed as the ratio between LD50 and ED50. Compounds which exhibit high therapeutic indices are preferred. The exact formulation, route of administration and dosage can be chosen by the individual physician in view of the patient's condition (see e.g. Fingl et al., 1975, in "The Pharmacological Basis of Therapeutics", Ch. 1, p. 1). Dosage amount and interval may be adjusted individually to provide plasma levels of the active moiety which are sufficient to maintain the receptor modulating effects, or minimal effective concentration (MEC). The MEC will vary for each compound but can be estimated from in vitro data, e.g. the concentration necessary to achieve a 50-90% inhibition of the receptor using the assays described herein. Compounds should be administered using a regimen which maintains plasma levels above the MEC for 10-90% of the time, preferably between 30-90% and most preferably between 50-90%. Dosages necessary to achieve the MEC will depend on individual characteristics and route administration. In cases of local administration or selective uptake, the effective local concentration of the drug may not be related to plasma concentration.

The actual amount of composition administered will, of course, be dependent on the subject being treated, on the subject's weight, the severity of the affliction, the manner of administration and the judgement of the prescribing physician. For antibodies or therapeutically active nucleic acid molecules, and other compounds e.g. a daily dosage of 0,001 to 100 mg/kg, particuarly 0,01 to 10 mg/kg per day is suitable.

Suitable routes of administration may, for example, include oral, rectal, transmucosal or intestinal administration; parenteral delivery, including intramuscular, subcutaneous, intramedullary injections, as well as intrathecal, direct intraventricular, intravenous, intraperitoneal, intranasal or intraocular injections.

Alternatively, one may administer the compound in a local rather than a systematic manner, for example, via injection of the compound directly into a solid tumour, often in a depot or sustained release formulation. Furthermore, one may administer the drug in a targeted drug delivery system, for example in a liposome coated with a tumour-specific antibody, The liposomes will be targeted to and taken up selectively by the tumour.

As outlined above, the present invention is particuarly suitable for the treatment or prevention of therapy-resistant hyperproliferative disorders, preferably therapy-resistant types of cancer, e.g. irradiation and/or medicament resistant types of cancer.

Treatment of cancer with irradiation and/or cytostatic and/or cytotoxic agents has been shown to activate stress kinase p38. Surprisingly, it was found that inhibition of receptor tyrosine kinase ligands such as HB-EGF strongly enhances the therapeutic activity of irradiation and/or chemotherapeutics, particuarly the apoptosis-inducing activity thereof. Thus, co-application of a direct receptor tyrosine kinase ligand inhibitor or inhibitors which prevent ligand precursor shedding with a further therapeutic procedure and/or medicament results in a substantive increase in sensitivity of the disorder against application of said further procedure and/or medicament and thus enhancement of the efficacy of said further therapeutic procedure and/or medicament. The administration if ligand inhibitors as described above is particularly suitable for the treatment or prevention of disorders which are at least partially resistant against irradiation therapy and/or administration of cytostatic and/or cytotoxic medicaments, particularly which are at least partially resistant against apoptosis-inducing procedures and medicaments.

In a preferred embodiment of the present invention the receptor tyrosine kinase ligand inhibitor is co-applied with an irradiation therapy, particuarly a gamma irradiation therapy. In a further preferrred embodiment the receptor tyrosine kinase ligand inhibitor is co-applied with a further anti-cancer medicament, particuarly an apoptosis-inducing medicament. Preferred examples of suitable anti-cancer medicaments are doxorubicin, taxanes, cis/trans-platin or derivatives thereof, 5-fluorouracil, mitomycin D, paclitaxel, etoposide, cyclophosphoamide, docetaxel or other apoptosis-inducing drugs or proteins, such as antibodies.

Co-application of the ligand inhibitor and the further procedure and/or medicament may be carried out simultaneously and/or sequentially. Application of the ligand inhibitor leads to an increased sensitivity of the disorder to be treated against application of other therapies. Particularly, tumour resistance against irradiation and/or chemotherapeutics is reduced.

Thus, a further aspect of the present invention is a pharmaceutical composition or kit comprising as active ingredients
(a) an inhibitor of a receptor tyrosine kinase ligand which is an inhibitor of a metalloprotease capable of cleaving the receptor tyrosine kinase ligand or an inhibitor of regulatory steps upstream of the metalloprotease,
   and
(b) a further medicament for the treatment of hyperproliferative disorders.

Preferably, the composition or kit additionally comprises pharmaceutically acceptable carriers, diluents and/or adjuvants.

Furthermore, a method is provided which allows for identifying modulators of stress-induced receptor tyrosine kinase activity e.g. p38-induced activity, comprising determining if a test compound is capable of inhibiting the activity of a ligand of said receptor tyrosine kinase. This method is suitable as a screening procedure, e.g. a high-through put screening procedure for identifying novel compounds or classes of compounds which are capable of modulating stress induced signal transduction. Further, the method is suitable as a validation procedure for characterizing the pharmaceutical efficacy and/or the side effects of compounds. The method may comprise the use of isolated proteins, cell extracts, recombinant cells or transgenic non-human animals. The recombinant cells or transgenic non-human animals preferably exhibit an altered metalloprotease and/or ligand expression compared to a corresponding wild-type cell or animal.

Furthermore, the invention shall be explained by the following figures and examples.

### Legends to Figures

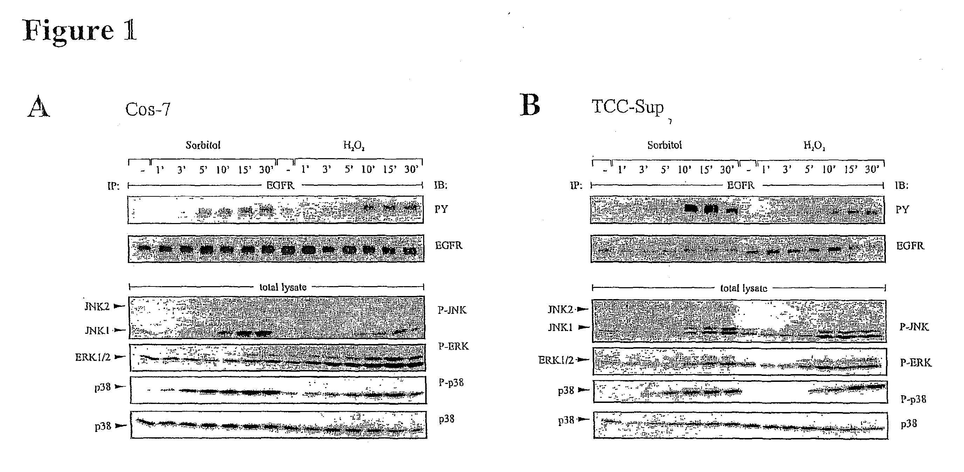

**Fig. 1****.** Time-course of stress-induced EGFR and MAPK phosphorylation in different cell lines. (A) EGFR and MAPK phosphorylation in response to osmotic and oxidative stress. Cos-7 cells were treated with sorbitol (0.3 M) and hydrogen peroxide (200 µM) for the indicated time periods. Following immunoprecipitation (IP) of cell extracts with anti-EGFR antibody proteins were immunoblotted (IB) with anti-phosphotyrosine antibody and re-probed with anti-EGFR antibody. Phosphorylated MAPKs were detected by immunoblotting total lysates with anti-phospho-ERK, anti-phospho-JNK and anti-phospho-p38 antibody. The same filters were re-probed with anti-p38 antibody. (B) Stress-induced EGFR phosphorylation in human bladder carcinoma cell lines. TCC-Sup cells were treated as indicated in (A).

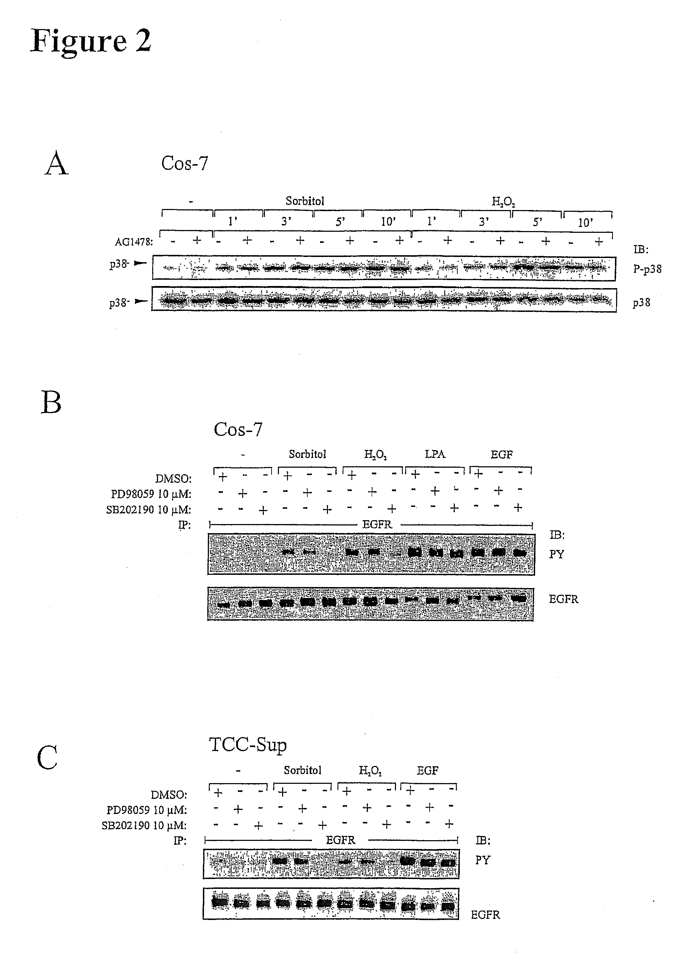

**Fig. 2****.** p38 mediates stress-induced EGFR phosphorylation. (A) Time-coure of p38 phosphorylation dependent on EGFR kinase function. Cos-7 cells were pretreated with AG1478 (250 nM) or an equal volume of empty vehicle (DMSO) for 20 min and stimulated with 0.3 M sorbitol or 200 µM hydrogen peroxide for the indicated periods. Cell extracts were immunoblotted with anti-phospho-p38 antibody and reprobing of the same filters with polyclonal anti-p38 antibody. (B) Stress-induced EGFR activation depends on p38 but not ERK activity in Cos-7 cells. Cos-7 cells were pre-treated with PD98059 (50 µM), SB202190 (10 µM) or an equal volume of empty vehicle (DMSO) for 30 min and stimulated with 0.3 M sorbitol and 200 µM hydrogen peroxide for 10 min and the GPCR agonist LPA (10 µM) or EGF (2 ng/mL) for 3 min as positive controls. Cell extracts were assayed for EGFR tyrosine phosphorylation content. (C) Stress-induced EGFR activation depends on p38 activity in TCC-Sup carcinoma cells. TCC-Sup cells were pretreated as described under (A) and stimulated with 0.3 M sorbitol and 200 µM hydrogen peroxide for 10 min and EGF (2 ng/mL) for 3 min as a positive control. After lysis cell extracts were assayed for EGFR tyrosine phosphorylation content.

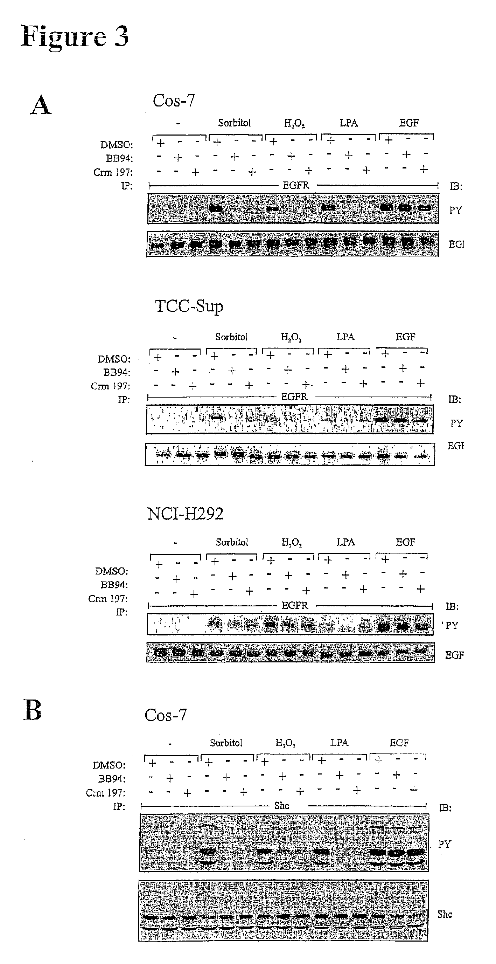

**Fig. 3****.** EGFR activation in response to osmotic and oxidative stress is ligand-dependent. (A) Effect of metalloprotease and HB-EGF inhibition on EGFR phosphorylation. Cos-7, NCI-H292 and TCC-Sup cells were serum-starved for 24 hrs, pre-treated with BB94 (10 µM), the diphtheria toxin mutant Crm197 (10 µg/mL) or an equal volume of empty vehicle (DMSO) for 20 min, and stimulated for 10 min with 0.3 M sorbitol, 200 µM hydrogen peroxide, 10 µM LPA or 2 ng/mL EGF. Following immunoprecipitation of cell extracts with anti-EGFR antibody proteins were immunoblotted with anti-phosphotyrosine antibody and re-probed with anti-EGFR antibody. (B) Analysis of Shc phosphorylation in response to stress agents. Cos-7 cells were treated as described under (A). After immunoprecipitation of Shc from cell extracts with a polyclonal anti-Shc antibody proteins were immunoblotted with anti-phosphotyrosine antibody and re-probed with anti-Shc antibody.

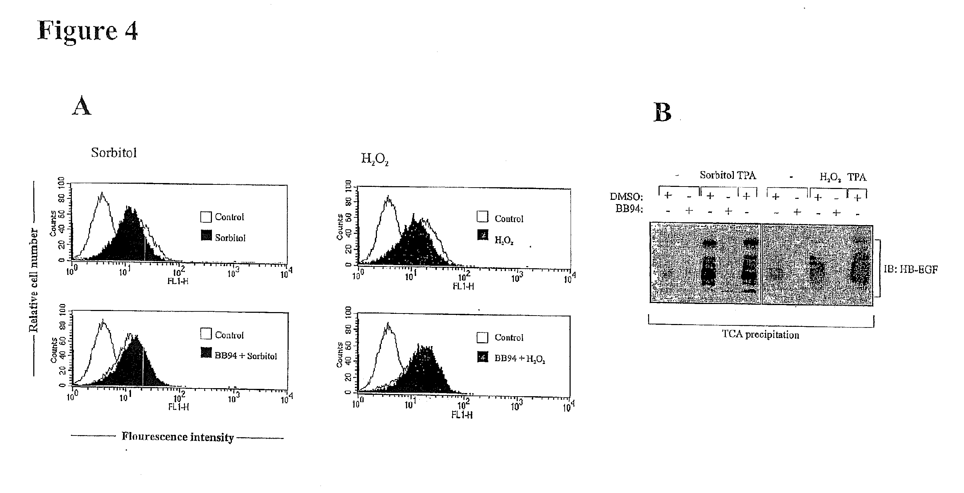

**Fig. 4****.** Analysis of proHB-EGF release in response to stress agents. (A) Flow cytometric analysis of proHB-EGF processing. Cos-7 cells were pretreated with BB94 (10 µM) or an equal volume of empty vehicle (DMSO) for 20 min, and stimulated with 0.3 M sorbitol or 200 µM hydrogen peroxide for 30 min. Cells were collected and stained for surface proHB-EGF and analyzed by flow cytometry. (B) Immunoblot analysis of conditioned media. Cos-7 cells were transiently transfected with proHB-EGF cDNA. After serum starvation for 24 hours cells were stimulated for 20 minutes with sorbitol (0.3 M) or hydrogen peroxide (200 µM), and proteins within the supernatant medium were precipitated using trichloric acid (TCA) precipitation.
Precipitated proteins were subjected to tricin-sodiumdodecylsulfafie gel electrophoresis following the protocol of Schägger-Jagow and subsequent immunoblot analysis with anti-HB-EGF antibody. TPA stimulation has been included as a positive control.

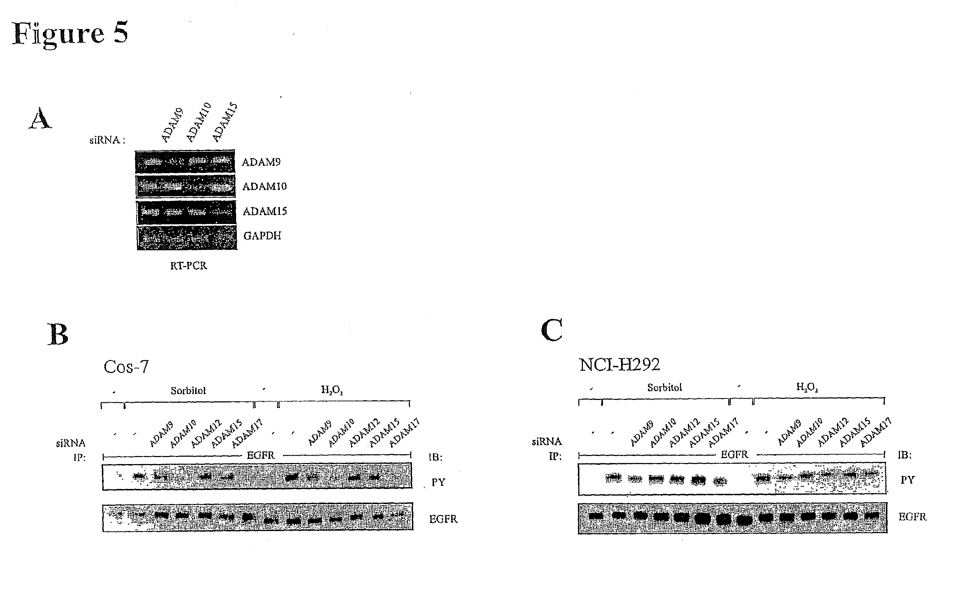

**Fig. 5****.** Effect of siRNAs against different ADAM proteins on stress-induced EGFR phosphorylation. (A) Blockade of ADAM metalloprotease expression by RNA interference (RNAi). NCI-H292 cells were transfected with siRNA against ADAM9, ADAM10 or ADAM15, cultured for 2 days and analyzed for gene expression by RT-PCR as indicated. (B) Cos-7 cells were transfected with siRNAs against ADAM9, -10, -12, -15 and -17, serum-starved for 24 hours, stimulated with 0.3 M sorbitol or 200 µM hydrogen peroxide for 10 min and assayed for EGFR tyrosine phosphorylation content. (C) NCI-H292 cells were treated as described under (B).

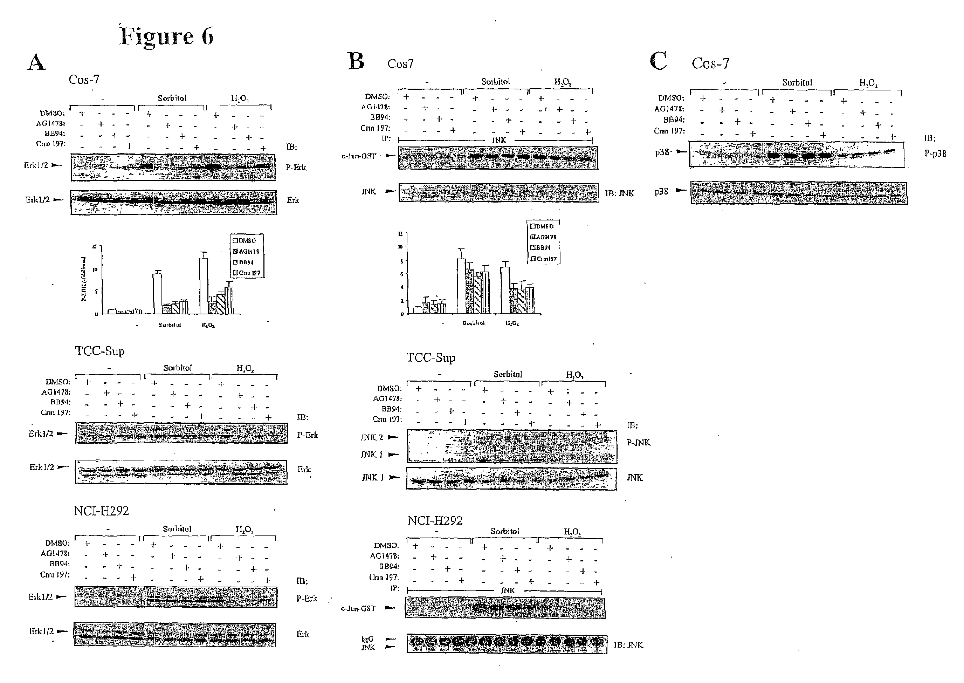

**Fig. 6****.** MAPK activation in response to stress agents and blockade of EGFR, metalloprotease and HB-EGF function. (A) Cos-7 cells transiently transfected with pcDNA3-HA-ERK2, TCC-Sup and NCI-H292 cells were pretreated with AG1478 (250 nM), BB94 (10 µM), Crm197 (10 µg/mL) or an equal volume of empty vehicle (DMSO) for 20 min and stimulated with 0.3 M sorbitol or 200 µM hydrogen peroxide for 30 min. After cell lysis total lysates were immunoblotted with anti-phospho-ERK antibody, followed by reprobing of the same membranes with polyclonal anti-ERK antibody. Quantitative analysis of ERK phosphorylation from three independent experiments (mean □ s.d.) using the FUJI LAS1000 imaging system. (B) Cos-7 and NCI-H292 cell were treated as described under (A). After lysis, JNK was immunoprecipitated using an anti-JNK antibody, and JNK activity was assayed using GST-c-JUN fusion protein as a substrate. Phosphorylated GST-c-JUN was visualized by autoradiography and JNK was immunoblotted in parallel using polyclonal JNK antibody. Quantitative analysis of GST-c-JUN phosphorylation from three independent experiments (mean □ s.d.) using the FUJI LAS1000 imaging system. TCC-Sup cells were treated as described under (A). After cell lysis, JNK phosphorylation was assayed by immunoblotting cell extracts with anti-phospho-JNK antibody and reprobing of the same filters with anti-JNK antibody. (C) p38 phosphorylation is independent of EGFR, metalloprotease and HB-EGF function. Cos-7 cells were treated as described under (A). p38 phosphorylation was assayed by immunoblotting cell extracts with anti-phospho-p38 antibody and re-probing the same filters with anti-p38 antibody.

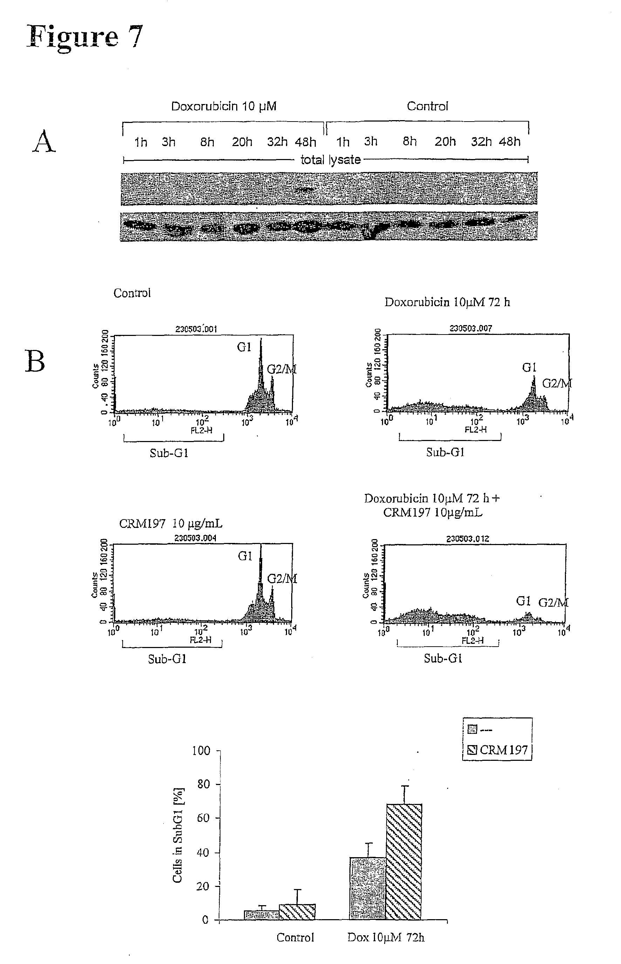

**Fig. 7****.** Doxorubicin-induced cell death of TCC-Sup carcinoma cells. (A) p38 activation in response to doxorubicin treatment. TCC-Sup cells were seeded and treated with doxorubicin for the indicated time points. After cell lysis, p38 activation was assessed by immunoblotting cell extracts with anti-phospho-p38 antibody and reprobing of the same filters with anti-p38 antibody. (B) Blockade of HB-EGF function enhances cell-death in response to doxorubicin. Cells were treated for 72 h with 10 µM doxorubicin and 10 µg/mL Crm197 every 24 h as indicated. After collection of cells in assay buffer, nuclei were stained with PI and analyzed by flow cytometric analysis.

### Examples

### 1. Materials and methods

### 1.1 Cell culture, plasmids and transfections

All cell lines (American Type Culture Collection, Manassas, VA) were routinely grown according to the supplier's instructions. Transfections of Cos-7 cells were carried out using Lipofectamine (Invitrogen) according to the manufacturer's protocol. Briefly, for transient transfections in 6 cm dishes cells were incubated for 4 h in 2 mL of serum-free medium containing 20 µL Lipofectamine and 2 µg of total plasmid DNA per dish. The transfection mixture was then supplemented with an equal volume of 20% fetal bovine serum and, 20 h later, cells were washed and cultured in serum-free medium for another 24 h prior to stimulation. The inhibitors AG1478 (Alexis Biochemicals), Batimastat (BB94, British Biotech, Oxford, UK), Crm197 (Quadratech Ltd., UK), SB202190 (Calbiochem) and PD98059 (Alexis Biochemicals) were added to serum-starved cells before the respective stimulation.

Dominant negative protease constructs of ADAM10, 12, 15 and 17 lacking the pro- and metalloprotease domains were previously described (Gschwind et al., 2003). The plasmids pcDNA3-HA-ERK2 (Daub et al., 1997) and pcDNA3-proHB-EGF-VSV (Prenzel et al., 1999) were used in this study.

### 1.2 Protein analysis

Cells were lysed and proteins immunoprecipitated as described before (Daub et al., 1997). Prior to lysis, cells grown to 80% confluence were treated with inhibitors and agonists as indicated in the figure legends and then lysed for 10 min on ice in buffer containing 50 mM HEPES, pH 7.5, 150 mM NaCl, 1% Triton X-100, 1 mM EDTA, 10% glycerol, 10 mM sodium pyrophosphate, 2 mM sodium orthovanadate, 10 mM sodium fluoride, 1 mM phenylmethylsulfonyl fluoride, and 10 µg/mL aprotinin. Lysates were precleared by centrifugation at 13,000 rpm for 10 min at 4°C. Precleared lysates were immunoprecipitated using the respective antibodies and 20 µL of protein A-Sepharose for 4 h at 4°C. Precipitates were washed three times with 0.5 mL of HNTG buffer, suspended in 2x SDS sample buffer, boiled for 3 min, and subjected to gel electrophoresis. Following SDS-polyacrylamide gel electrophoresis, proteins were transferred to nitrocellulose membrane. Western blots were performed according to standard methods. The antibodies against human EGFR (108.1) and SHC have been characterized before (Prenzel et al., 1999). Phosphotyrosine was detected with the 4G10 monoclonal antibody (UBI, Lake Placid, NY). Polyclonal anti-phospho-p44/p42 (Thr202/Tyr204) MAPK antibody, and anti-phospho-JNK (Thr183/Tyr185) and anti-phospho-p38 (Thr180/Tyr182) antibody were purchased from New England Biolabs (Beverly, MA). Polyclonal anti-ERK2, anti-JNK1 and anti-p38 antibody was from Santa Cruz Biotechnology (Santa Cruz, CA).

### 1.3 JNK activity assay

JNK activity was assayed as described previously (Sudo and Karin, 2000). Cultured cells were lysed in lysis buffer containing 20 mM Tris (pH7.6), 0.5% Nonidet P-40, 250 mM NaCl, 3 mM EDTA, 1 mM dithiotreitol, 0.5 mM phenylmethylsulfonylfluoride, 20 mM β-gylcerophosphate, 1 mM sodium orthovanadate and 1µg/mL leupeptin. JNK was immunoprecipitated from lysates obtained from 6-well dishes using polyclonal anti-JNK antibody. Immunoprecipitates were washed twice using lysis buffer and twice using kinase assay buffer (25 mM HEPES (pH 7.5), 20 mM β-gylcerophosphate, 20 mM PNPP, 20 mM MgCl₂, 2 mM dithiotreitol and 0.1 mM sodium orthovanadate). Kinase reactions were performed in 30 µL of kinase buffer supplemented with 1 µg GST-c-Jun (aa1-79), 20 µM cold ATP and 5 µCi of [y-³²P]ATP at 30° C for 30 minutes. Reactions were stopped by addition of 30 µL of Laemmli buffer and subjected to gel electrophoresis on 12.5% gels. Labeled GST-c-Jun was quantitated using a Phosphoimager (Fuji).

### 1.4 Flow cytometric analysis

FACS analysis was performed as described before (Prenzel et al., 1999). In brief, cells were seeded, grown for 20 h and serum-starved for 24 h. Cells were treated with inhibitors and stimulated as indicated. After collection, cells were stained with an ectodomain-specific antibody against proHB-EGF for 45 min. After washing with PBS, cells were incubated with FITC-conjugated secondary antibodies for 15 min and washed again with PBS. Cells were analysed on a Becton Dickinson FACScalibur flow cytometer.

### 1.5 TCA precipitation of HB-EGF

Cos-7 transiently transfected with pcDNA3-proHB-EGF-VSV were serum-starved for 24 h. Prior to stimulation cells were washed, preincubated with BB94 (10 µM) and stimulated as indicated. After stimulation the supernatant was collected, sodium-desoxycholate was added (100 µg/mL) and following incubation on ice for 10 minutes the solution was supplemented with trichloro acetic acid (TCA) to a final concentration of 10% TCA. After incubation on ice for 30 minutes samples were centrifuged, the supernatant was discarded and the precipitates were resuspended in Schägger-Jargow sample-buffer. TCA was neutralized using Tris-HCl (pH 8.8), and samples were separated using the tricine-SDS gel electrophoresis protocol (Schägger and von Jargow, 1987).

1.6 *RNA interference and RT-PCR analysis* Transfection of 21-nucleotide siRNA duplexes (Dharmacon Research, Lafayette, CO, USA) for targeting endogenous genes was carried out using Oligofectamine (Invitrogen) for NCI-H292 cells and 4.2 µg siRNA duplex per 6-well plate as previously described (Elbashir et al., 2001). Cos-7 cells were transfected using Lipofectamine 2000 (Invitrogen) according to the manufacturer's protocol. Briefly, 8.4 µg siRNA duplex per 6 cm dish were incubated with 10 µL Lipofectamine 2000 in 1 mL serum-free medium for 20 minutes. The transfection mixture was added to the cell culture medium containing serum and, after 6 h, cells were washed and incubated in medium containing serum overnight. NCI-H292 and Cos-7 cells were serum-starved and assayed 2 d after transfection. Highest efficiencies in silencing target genes were obtained by using mixtures of siRNA duplexes targeting different regions of the gene of interest. Sequences of siRNA used were SEQ ID NO 1: AAUCACUGUGGAGACAUUUGCdTdT,

SEQ ID NO. 2: AAACUUCCAGUGUGUAGAUGCdTdT (ADAM9);

SEQ ID NO 3: AAUGAAGAGGGACACUUCCCUdTdT,

SEQ ID NO 4: AAGUUGCCUCCUCCUAAACCAdTdT (ADAM10);

SEQ ID NO 5: AACCUCGCUGCAAAGAAUGUGdTdT,

SEQ ID NO 6: AAGACCUUGATACGACUGCUGdTdT (ADAM12);

SEQ ID NO 7: AACUCCAUCUGUUCUCCUGACdTdT,

SEQ ID NO 8: AAAUUGCCAGCUGCGCCCGUCdTdT (ADAM15);

SEQ ID NO 9: AAAGUUUGCUUGGCACACCUUdTdT,

SEQ ID NO 10: AAGUAAGGCCCAGGAGUGUUdTdT,

SEQ ID NO 11: AACAUAGAGCCACUUUGGAGAdTdT (ADAM17);

SEQ ID NO 12: CGUACGCGGAAUACUUCGAdTdT (control, GL2).

The siRNA-duplexes against ADAM12 and ADAM17 have been described earlier (Gschwind et al., 2003).

Specific silencing of targeted genes was confirmed by RT-PCR analysis. RNA isolated using RNeasy Mini Kit (Qiagen, Hilden, Germany) was reverse transcribed using AMV Reverse Transcriptase (Roche, Mannheim, Germany). PuReTaq Ready-To-Go PCR Beads (Amersham Biosciences, Piscataway, NJ) were used for PCR amplification. Primers (Sigma Ark, Steinheim, Germany) were

ADAM9,SEQ ID NO 13: 5'-AGT GCA GAG GAC TTT GAG AA-3' and SEQ

ID NO 14:5'-TGC CGT TGT AGC AAT AGG CT-3',

ADAM10, SEQ ID NO 15: 5'-TTG CTC ACG AAG TTG GAC AT-3' and SEQ

ID NO 16:5'-TTT CCC AGG TTT CAG TTT GC-3',

SEQ ID NO 17:ADAM15, 5'-GGC TGG CAG TGT CGT CCT ACC AGA
GGG-3' and SEQ ID NO 18:5'-GGT GCA CCC AGC TGC AGT TCA GCT
CAG TCC-3'.

PCR products were subjected to electrophoresis on a 2.5% agarose gel and DNA was visualized by ethidium bromide staining.

1.7 *Apoptosis Assay* TCC-Sup bladder carcinoma cells were seeded, grown for 20 h and treated with 10 µM doxorubicin and Crm197 as indicated for 72 h. Cells were collected in assay buffer containing propidium iodide (PI), and incubated at 4° C for 3 h. Nuclear PI staining was analysed on a Becton Dickinson FACScalibur flow cytometer.

### 2. RESULTS

### 2.1 Distinct kinetics of EGFR and MAPK activation in Cos-7 and human carcinoma cell lines

Osmotic and oxidative stress lead to phosphorylation of the EGFR and MAPKs in a wide variety of cell systems (Carpenter, 1999; de Nadal et al., 2002; King et al., 1989). To investigate the underlying mechanisms we performed time course experiments in Cos-7 and TCC-Sup bladder carcinoma cell lines by immunoblot analysis. As shown in Figure 1 (upper panel), tyrosine phosphorylation of the EGFR in response to the stress agents sorbitol (0.3 M) and hydrogen peroxide (200 µM) occurred in both cell lines within 5 to 10 min, while the MAPKs ERK1/2 and JNK became phosphorylated after 10 and 15 min, respectively (Fig. 1A/B, lower panel). A weak activation of ERK1/2 was already detectable after 1 min, while the phosphorylation is markedly increased after 10 min. In contrast, phosphorylation of p38 occurred as an immediate response to stress agents. The results of these time course experiments suggested that the EGFR might be involved in ERK1/2 and JNK activation in response to stress stimuli. In contrast, activation of p38 preceded EGFR stimulation. Interestingly, receptor activation was detectable on a timescale that not only allows ligand-independent but also ligand-dependent activation mechanisms.

### 2.2 p38 controls EGFR activation by osmotic and oxidative stress.

The finding that p38 activation preceded EGFR tyrosine phosphorylation (Fig. 1) raised the question whether p38 acts as an upstream regulator of EGFR stimulation in Cos-7 and human carcinoma cell lines.

Preincubation of Cos-7 cells with the selective EGFR-kinase inhibitor AG1478 did not affect p38 phosphorylation in response to stress agents (Fig. 2A), demonstrating that p38 activation occurred independent of EGFR activity. To address the question whether p38 is located upstream of the EGFR we used the p38-specific inhibitor SB202190 to investigate the effect of blocking p38 activity on stress-induced EGFR activation. As shown in Figure 2B, preincubation of Cos-7 cells with SB202190 completely abrogated the stress-induced EGFR activation while leaving lysophosphatidic acid (LPA) and EGF-induced receptor phosphorylation unaffected. In contrast, the MEK1/2 inhibitor PD98059 did not affect EGFR phosphorylation. Similar results were obtained in the bladder carcinoma cell line TCC-Sup (Fig. 2C). Taken together, these results identify p38 as an upstream mediator of stress-induced EGFR activation in Cos-7 and TCC-Sup bladder carcinoma cells.

### 2.3 Stress-induced EGFR phosphorylation depends on metalloprotease activity and HB-EGF function.

Recent investigations underlined the importance of EGF-like ligand proteolysis in EGFR signalling, especially in signal transduction events that were previously thought to be ligand-independent such as EGFR signal transactivation by GPCR (Prenzel et al., 1999). Based on these findings we adressed the question whether EGFR activation by stress stimuli can also involve a ligand-dependent mechanism. Therefore, cells were preincubated with the metalloprotease inhibitor batimastat (BB94) that has been shown to inhibit EGF-like ligand processing and subsequent EGFR transactivation (Prenzel et al., 1999). EGFR tyrosine phosphorylation was monitored after stimulation with stress agents using immunoblot analysis. As shown in Figure 3A, BB94 almost completely blocked sorbitol or hydrogen peroxide-induced EGFR phosphorylation in Cos-7 and TCC-Sup bladder carcinoma cells (upper and middle panel). In the lung carcinoma cell line NCI-H292 BB94 inhibited EGFR activation to 50%, suggesting an alternative parallel activation mechanism (Fig. 3A, lower panel).

Next, we investigated the effect of the diphtheria toxin mutant Crm197 which specifically blocks HB-EGF function on stress-activated EGFR tyrosine phosphorylation. Indeed, Crm197 inhibited EGFR phosphorylation to the same extent as BB94, suggesting that in these three cell lines HB-EGF is critically involved in stress-induced EGFR activation. As a positive control receptor activation by LPA was completely prevented by these inhibitors while direct stimulation with EGF was unaffected.

Furthermore, it was of special interest whether this ligand-dependency can be followed to downstream signalling partners of the EGFR. Shc adaptor proteins are prominent signalling adaptors of the EGFR linking the receptor to activation of the Ras/Raf/ERK-MAPK cascade. Indeed, the data shown in Figure 3B (lower panel) demonstrate that the phosphorylation content of Shc proteins in response to stress stimuli resembles that of the EGFR itself. Therefore, phosphorylation of Shc by stress stimuli critically depends on a ligand-dependent EGFR phosphorylation mechanism.

### 2.4 Ectodomain shedding of proHB-EGF is induced in response to osmotic and oxidative stress in Cos-7 cells.

To further substantiate the role of HB-EGF in this ligand-dependent EGFR stimulation mechanism we investigated shedding of proHB-EGF in response to hyperosmolarity and hydrogen peroxide treatment on the level of the ligand itself. Therefore, we analyzed the amount of proHB-EGF present on the cell surface of Cos-7 cells prior to and after stimulation with sorbitol or hydrogen peroxide by flow cytometric analysis. As shown in Figure 4A, both stimuli lead to a significant decrease of the HB-EGF precursor on the cell surface. Moreover, in accordance with the results presented in Figure 3A, pre-treatment with the metalloprotease inhibitor BB94 abolished proHB-EGF processing. In addition to the decrease of HB-EGF precursor, we determined the amount of mature soluble HB-EGF released from the cell surface of Cos-7 cells ectopically expressing proHB-EGF into the cell culture medium. As shown in Figure 4B, both, treatment with hyperosmolarity and hydrogen peroxide, induced an increase of HB-EGF in the conditioned medium as determined by immunoblot analysis. Again, HB-EGF release was blocked by preincubation with the metalloprotease inhibitor BB94 confirming the involvement of a metalloprotease activity.

### 2.5 Metalloproteases of the ADAM family mediate EGFR activation by osmotic and oxidative stress.

The finding that metalloprotease-dependent mechanisms significantly contribute to stress-induced EGFR and Shc activation raised the question which metalloprotease(s) are involved. We used the RNA interference technique to inhibit the endogenous expression of the ADAM proteases ADAM9, -10, -12, -15 and ADAM17 that have already been implicated in EGF-like ligand cleavage. Figure 5A demonstrates the efficient and specific knockdown of target gene expression by the siRNAs against ADAM9, 10 and 15 using RT-PCR. The siRNAs against ADAM12 and 17 have been described earlier (Gschwind et al., 2003). Transient transfection with these siRNAs directed against the respective protease and subsequent immunoblot analysis of the EGFR after stimulation with sorbitol or hydrogen peroxide revealed that ADAM10 and ADAM17 are involved in EGFR activation after both stress stimuli in Cos-7 cells (Fig. 5B). However, since we were interested in the regulation of these processes in human cancer cells we further investigated the involvement of ADAM family members in the lung carcinoma cell line NCl-H292: similar to the results obtained in Cos-7 cells ADAM17 is also involved in stress-induced EGFR activation as demonstrated in Figure 5C. In contrast to Cos-7 cells, ADAM9 and in the case of hydrogen peroxide treatment also ADAM12 participate in the stress response of NCI-H292 cells. Taken together, depending on the cell type different members of the ADAM family of metalloproteases, particularly ADAM17, play a central role in HB-EGF-dependent EGFR activation in response to stress agents.

### 2.6 Activation of the MAPKs ERK1/2 and JNK in response to hyperosmolarity and oxidative stress is mediated by HB-EGF-dependent EGFR activation.

Since the MAPKS ERK1/2 and JNK are activated by hypertonicity and reactive oxygen species (de Nadal et al., 2002; Kyriakis and Avruch, 2001), we asked whether the ligand-dependent EGFR phosphorylation contributes to the induction of MAPK activation by stress stimuli. Therefore, we used the selective EGFR tyrphostin AG1478 to investigate the overall dependence of stress-induced MAPK activation on the EGFR kinase activity. Furthermore, we compared this effect with inhibition of the ligand-dependent EGFR activation process by BB94 and Crm197. As shown in Figure 6A (upper panel), the sorbitol and hydrogen peroxide-induced activation of ERK1/2 is blocked by AG1478. In addition, BB94 and Crm197 are almost as effective in blocking ERK1/2 phosphorylation. These data suggest that ERK1/2 activation in Cos-7 cells in response to osmotic and oxidative stress almost completely depends on EGFR activation which can be largely attributed to a ligand-dependent mechanism. The same experimental setup was used to address the question whether this mechanistic concept can be extended to stress signalling in human carcinoma cell lines. However, we found that hyperosmolarity and oxidative stress-induced ERK1/2 activation in TCC-Sup and NCl-H292 cells was substantially blocked by AG1478, BB94 and Crm197 (Fig. 6A, middle and lower panel). Interestingly, although oxidative stress induces EGFR phosphorylation only partially through a ligand-dependent mechanism in NCI-H292 cells, ERK activation largely depends on proHB-EGF processing in this cell type.

Apart from ERK1/2, we were interested in the signalling mechanisms leading to activation of JNK. Figure 6B (upper panel) shows the JNK activity induced by osmotic and oxidative stress depending on treatment with AG1478, BB94 and Crm197. While in Cos-7 cells sorbitol-induced JNK activity is largely independent of the EGFR, JNK activity in response to oxidative stress depends to 50% on EGFR activation via HB-EGF. Similar results were obtained in the bladder carcinoma cell line TCC-Sup as shown in Figure 6B (middle panel). In contrast, hydrogen peroxide stimulated JNK activation in the lung carcinoma cell line NCI-H292 appears to be independent of the EGFR, while JNK activity in response to hypertonicity partially depends on a ligand-dependent EGFR activation pathway. In accordance with the finding that p38 acts as an upstream mediator of stress-induced EGFR activation (Fig. 2) p38 activity neither depends on the EGFR kinase activity nor on metalloprotease or HB-EGF function (Fig. 6C).

Treatment of tumour cells with chemotherapeutics has been shown to activate the stress kinases p38 and JNK leading to cell death. However, as p38 activation leads to HB-EGF-dependent EGFR- and Erk1/2 activation, we investigated the effect of blocking HB-EGF function on the doxorubicin-induced apoptosis of TCC-Sup bladder carcinoma cells. Fig. 7A demonstrates that doxorubicin treatment induces p38 activation. Interestingly, Fig. 7B/C show that treatment of TCC-Sup cells with the HB-EGF inhibitor CRM197 strongly enhances the apoptotic response to doxorubicin when compared to doxorubicin alone, while treatment with CRM197 alone exerts only minor effects.

Together, these data demonstrate that ligand-dependent EGFR tyrosine phosphorylation by stress agents plays a critical role in the activation of the MAPKs ERK1/2 and JNK in response to osmotic and oxidative stress in human cancer cells. Moreover, these results implicate the herein presented signalling mechanism as a pathway employed by tumour cells to evade chemotherapy-induced cell death.

### 3. DISCUSSION

Intensive investigations addressed the question of how mammalian cells deal with stress agents. Our present study provides new insights in growth factor-dependent mechanisms leading to EGFR and subsequent MAPK activation in response to osmotic and oxidative stress in human carcinoma cells.

We have demonstrated here that EGFR phosphorylation induced by both osmotic and oxidative stress requires a metalloprotease activity and release of HB-EGF in Cos-7 cells and human carcinoma cell lines. This result was obtained both on the level of the EGFR (Fig. 3) and of the EGF-like ligand HB-EGF (Fig. 4). Previous reports demonstrated that hyperosmolarity and oxidative stress may lead to EGFR activation by receptor aggregation and dimerization (Rosette and Karin, 1996). Alternatively, inactivation of phosphatases has been proposed leading to stimulation of intracellular kinases or the receptor, respectively (Blanchetot et al., 2002; Knebel et al., 1996), thereby inducing EGFR phosphorylation. Within this context the finding that stress-induced EGFR phosphorylation in NCI-H292 cells partially depends on ligand-dependent and partially on ligand-independent mechanisms (Fig. 3) is in agreement with these previous investigations, as both mechanisms might cooperate in receptor activation. The respective contribution of these mechanisms is likely to depend on the cellular context, as in Cos-7 and TCC-Sup bladder carcinoma cells a ligand-dependent mechanism fully accounts for receptor phosphorylation by osmotic and oxidative stress (Fig. 3). Our results are further supported by previous investigations demonstrating that stress stimuli induce the expression of HB-EGF and amphiregulin in rat gastric epithelial cells (Miyazaki et al., 2001). Moreover, a recent report by Frank et al. (Frank et al., 2003) implicated HB-EGF in hydrogen peroxide-induced EGFR phosphorylation in vascular smooth muscle cells. Shedding of proTGF-α in response to hyperosmolarity has been reported in CHO cells, which lack endogenous EGFR (Fan and Derynck, 1999; Montero et al., 2002). A report by Chen et al. (Chen et al., 2001 a) attributed the hydrogen peroxide-induced EGFR activation in Cos-7 cells to a ligand-independent mechanism via c-Src. In contrast to this, we did not achieve specific inhibition of EGFR activation by oxidative stress with the Src inhibitors PP1 or PP2 in the same cell system (unpublished data).

Increasing evidence has implicated the ADAM family of zinc-dependent proteases as crucial mediators of EGF-like ligand processing. In accordance with these reports our results demonstrate that ADAM proteases are responsible for shedding of proHB-EGF induced by stress stimuli. Interestingly, while in the context of EGFR transactivation single distinct ADAM proteases are activated (Gschwind et al., 2003; Yan et al., 2002), we found that depending on the cellular system two or more ADAM proteases become active (Fig. 5). Importantly, ADAM17 appears to be generally involved in stress stimulated shedding events, while also ADAM9, ADAM10 and ADAM12 can be involved (Fig. 5). All of these enzymes have been previously implicated in EGF-like ligand shedding (Asakura et al., 2002; Izumi et al., 1998; Lemjabbar and Basbaum, 2002; Yan et al., 2002; Gschwind et al., 2003). The finding that different ADAM proteases become activated, but only proHB-EGF is cleaved appears surprising as amphiregulin and TGF-α are also expressed in NCI-H292 cells (data not shown). On the other hand our results are corroborated by previous reports demonstrating that ADAM9 cleaves proHB-EGF in response to TPA stimulation in VeroH cells (Izumi et al., 1998), while in the same cellular system proHB-EGF processing after LPA stimulation is independent of ADAM9 (Umata et al., 2001), suggesting that proHB-EGF sheddases are defined by both, the cellular context and stimulus. Moreover, ADAM9 knockout mice lack an obvious phenotype and ADAM9 ^{-/-} fibroblasts display no defects in proHB-EGF processing (Weskamp et al., 2002), strongly arguing for functional redundancy among proHB-EGF cleaving enzymes in vivo.

How are the metalloproteases of the ADAM family activated, finally leading to EGFR phosphorylation and downstream signalling responses? Previous reports demonstrated regulation of metalloprotease-mediated ectodomain cleavage of transmembrane proteins in response to growth factors and TPA by the MAPK ERK1/2, while the basal level of ectodomain shedding has been attributed to p38 activity (Fan and Derynck, 1999; Gechtman et al., 1999; Rizoli et al., 1999). Moreover, p38 has been implicated as an upstream mediator of the EGFR in the sorbitol-induced EGFR activation in human non-transformed keratinocytes (Cheng et al., 2002). In contrast to our results, the authors excluded a ligand-dependent mechanism based on medium transfer experiments. As the released EGF-like ligand may be retained in the extracellular matrix binding to heparan sulfate glycans involvement of ligand-dependent EGFR activation cannot be ruled out. These reports and the finding that p38 activity in our systems is independent of the EGFR phosphorylation state (Fig.2 and Fig. 6C) prompted us to ask the question if p38 is the upstream signalling element that controls ligand-dependent EGFR activation and downstream signalling by stress agents. Indeed, we found that preincubation with a specific p38 inhibitor abrogated stress-induced EGFR activation, while blocking ERK1/2 activation leaves EGFR phosphorylation unaffected (Fig. 2B). On the contrary, p38 activation itself in response to stress agents is independent of the EGFR as assessed using the EGFR selective inhibitor AG1478 (Fig. 2A). Furthermore, time course experiments revealed that p38 activation precedes EGFR phosphorylation, which is a necessary prerequisite for p38 being upstream located of the EGFR, while ERK1/2 and JNK activation occurs even later (Fig. 1). Together, these data suggest p38 as the upstream inducer of ligand-dependent EGFR activation and its subsequent downstream signalling.

This crucial role of p38 in the stress response is well conserved throughout evolution. In *Saccharomyces cerevisae* the response to hyperosmolarity has been intensively studied. A central role plays the kinase HOG, the yeast homolog of human p38. HOG is activated by sensor molecules for changing osmolarity that have no human counterparts. Activation of HOG by these proteins leads to adaptive responses in yeast to deal with hyperosmolarity (de Nadal et al., 2002).

Activation of ERK1/2 and JNK in response to oxidative and osmotic stress represents an important step in the cellular stress response (reviewed in Kyriakis and Avruch, 2001). While the phosphorylation of different receptor tyrosine kinases as potential mediators of MAPK signalling is stimulated by stress agents numerous reports reveal an outstanding role for the EGFR in MAPK activation by stress stimuli (reviewed in Carpenter, 1999). Recent investigations provide evidence for the severe consequences of stress signalling via MAPKs in anticancer therapy, as cancer cells frequently produce high levels of ROS (Burdon, 1995; Szatrowski and Nathan, 1991). Moreover, anticancer drugs or radiation therapy lead to activation of stress signalling cascades (Benhar et al., 2002), which has been attributed to the production of ROS caused by these agents. Furthermore, EGFR-dependent MAPK signalling has been reported to affect the expression levels of apoptosis regulators such as the Bcl-2 family (Jost et al., 2001). For these reasons elucidating stress signalling mechanisms and their complex interplay has gained increased attention due to the therapeutic implications.

Here, we show that the stress-induced ligand-dependent EGFR activation leads to downstream signalling events which depend in the case of ERK1/2 critically and in the case of JNK to a large degree on EGFR activation (Fig. 6). Previous reports implicated different ligand-independent activation mechanisms in stress-induced MAPK activation. These pathways involve the inactivation and downregulation of phosphatases and other redox-susceptible proteins, affecting scaffolding proteins and small G-proteins. Our data extend these results as they demonstrate that both EGF-like ligand-dependent and -independent mechanisms cooperate in MAPK activation (Fig. 6). The respective contribution of these pathways is likely to depend on the specific cellular context. Ligand-dependent EGFR activation appears to mediate cross-talk between different MAPK signalling pathways. Moreover, we demonstrated that doxorubicin-induced apoptosis in bladder carcinoma cells can be strongly enhanced by blockade of HB-EGF function (Fig 7), while doxorubicin treatment induces p38 activation. As chemotherapeutic agents have been previously shown to activate stress signalling cascades (Benhar et al., 2002), this signalling mechanism provides a molecular explanation for tumour cells evading drug-induced cell death.

To our knowledge this is the first report demonstrating the ligand-dependent activation of the EGFR and subsequent downstream signalling by osmotic and oxidative stress agents regulated by the MAPK p38 within human carcinoma cells. Increasing evidence implicates particularly oxidative stress caused by the excessive production of ROS in a variety of human disorders as diverse as cardiovascular, neurodegenerative or hyperproliferative diseases such as cancer. Therefore, our results are of special importance for pathophysiological disorders and the respective therapeutic approaches involving cellular damage caused by stress agents.

The data presented here extend previous results on the signalling mechanisms of stress stimuli in mammalian cells, particularly in human carcinomas. Our findings substantiate the importance of ADAM family proteases and HB-EGF as critical mediators of the stress response in human cancer cells. Furthermore, our data suggest that cross-communication between different groups of MAPK employs ADAM proteases and the EGFR as signalling intermediates. Within this context the balance between ERK1/2 and JNK activity is of special significance for the cell's fate. Future investigations will further have to focus on other EGFR downstream signalling events and possible pathobiological responses such as enhanced proliferation or migration of cancer cells in response to stress agents.

### References

Asakura, M., Kitakaze, M., Takashima, S., Liao, Y., Ishikura, F., Yoshinaka, T., Ohmoto, H., Node, K., Yoshino, K., Ishiguro, H., Asanuma, H., Sanada, S., Matsumura, Y., Takeda, H., Beppu, S., Tada, M., Hori, M. and Higashiyama, S. (2002) Cardiac hypertrophy is inhibited by antagonism of ADAM12 processing of HB-EGF: metalloproteinase inhibitors as a new therapy. Nat Med, 8, 35-40.

Benhar, M., Engelberg, D. and Levitzki, A. (2002) ROS, stress-activated kinases and stress signaling in cancer. EMBO Rep, 3, 420-425.

Biscardi, J.S., Maa, M.C., Tice, D.A., Cox, M.E., Leu, T.H. and Parsons, S.J. (1999) c-Src-mediated phosphorylation of the epidermal growth factor receptor on Tyr845 and Tyr1101 is associated with modulation of receptor function. J Biol Chem, 274, 8335-8343.

Blanchetot, C., Tertoolen, L.G.J. and den Hertog, J. (2002) Regulation of receptor protein-tyrosine phosphatase {alpha} by oxidative stress. EMBO J., 21, 493-503.

Borrell-Pages, M., Rojo, F., Albanell, J., Baselga, J. and Arribas, J. (2003) TACE is required for the activation of the EGFR by TGF-alpha in tumors. Embo J, 22, 1114-1124.

Burdon, R.H. (1995) Superoxide and hydrogen peroxide in relation to mammalian cell proliferation. Free Radic Biol Med, 18, 775-794.

Carpenter, G. (1999) Employment of the epidermal growth factor receptor in growth factor-independent signaling pathways. J Cell Biol, 146, 697-702.

Chen, K., Vita, J.A., Berk, B.C. and Keaney, J.F., Jr. (2001 a) c-Jun N-terminal kinase activation by hydrogen peroxide in endothelial cells involves SRC-dependent epidermal growth factor receptor transactivation. J Biol Chem, 276, 16045-16050.

Chen, Z., Gibson, T.B., Robinson, F., Silvestro, L., Pearson, G., Xu, B., Wright, A., Vanderbilt, C. and Cobb, M.H. (2001 b) MAP kinases. Chem Rev, 101, 2449-2476.

Cheng, H., Kartenbeck, J., Kabsch, K., Mao, X., Marques, M. and Alonso, A. (2002) Stress kinase p38 mediates EGFR transactivation by hyperosmolar concentrations of sorbitol. J Cell Physiol, 192, 234-243.

Daub, H., Wallasch, C., Lankenau, A., Herrlich, A. and Ullrich, A. (1997) Signal characteristics of G protein-transactivated EGF receptor. Embo J, 16, 7032-7044.

Daub, H., Weiss, F.U., Wallasch, C. and Ullrich, A. (1996) Role of transactivation of the EGF receptor in signalling by G-protein- coupled receptors. Nature, 379, 557-560. de Nadal, E., Alepuz, P.M. and Posas, F. (2002) Dealing with osmostress through MAP kinase activation. EMBO Rep, 3, 735-740.

Diaz-Rodriguez, E., Montero, J.C., Esparis-Ogando, A., Yuste, L. and Pandiella, A. (2002) Extracellular Signal-regulated Kinase Phosphorylates Tumor Necrosis Factor alpha -converting Enzyme at Threonine 735: A Potential Role in Regulated Shedding. Mol. Biol. Cell, 13, 2031-2044.

Fan, H. and Derynck, R. (1999) Ectodomain shedding of TGF-alpha and other transmembrane proteins is induced by receptor tyrosine kinase activation and MAP kinase signaling cascades. EMBO J., 18, 6962-6972.

Fan, H., Turck, C.W. and Derynck, R. (2003) Characterization of growth factor-induced serine phosphorylation of tumor necrosis factor-alpha converting enzyme (TACE) and of an alternatively translated polypeptide. J Biol Chem.

Finkel, T. (1998) Oxygen radicals and signaling. Curr Opin Cell Biol, 10, 248-253.

Frank, G.D., Mifune, M., Inagami, T., Ohba, M., Sasaki, T., Higashiyama, S., Dempsey, P.J. and Eguchi, S. (2003) Distinct mechanisms of receptor and nonreceptor tyrosine kinase activation by reactive oxygen species in vascular smooth muscle cells: role of metalloprotease and protein kinase C-delta. Mol Cell Biol, 23, 1581-1589.

Gechtman, Z., Alonso, J.L., Raab, G., Ingber, D.E. and Klagsbrun, M. (1999) The shedding of membrane-anchored heparin-binding epidermal-like growth factor is regulated by the Raf/mitogen-activated protein kinase cascade and by cell adhesion and spreading. J Biol Chem, 274, 28828-28835.

Gschwind, A., Hart, S., Fischer, O.M. and Ullrich, A. (2003) TACE Cleavage of Proamphiregulin Regulates GPCR-induced Proliferation and Motility of Cancer Cells. Embo J, In Press.

Gschwind, A., Prenzel, N. and Ullrich, A. (2002) Lysophosphatidic Acidinduced Squamous Cell Carcinoma Cell Proliferation and Motility Involves Epidermal Growth Factor Receptor Signal Transactivation. Cancer Res, 62, 6329-6336.

Izumi, Y., Hirata, M., Hasuwa, H., Iwamoto, R., Umata, T., Miyado, K., Tamai, Y., Kurisaki, T., Sehara-Fujisawa, A., Ohno, S. and Mekada, E. (1998) A metalloprotease-disintegrin, MDC9/meltrin-gamma/ADAM9 and PKCdelta are involved in TPA-induced ectodomain shedding of membrane-anchored heparin-binding EGF-like growth factor. Embo J, 17, 7260-7272.

Johnson, G.L. and Lapadat, R. (2002) Mitogen-Activated Protein Kinase Pathways Mediated by ERK, JNK, and p38 Protein Kinases. Science, 298, 1911-1912.

Jost, M., Huggett, T.M., Kari, C., Boise, L.H. and Rodeck, U. (2001) Epidermal growth factor receptor-dependent control of keratinocyte survival and Bcl-xL expression through a MEK-dependent pathway. J Biol Chem, 276, 6320-6326.

Kamata, H. and Hirata, H. (1999) Redox regulation of cellular signalling. Cell Signal, 11, 1-14.

Keates, S., Sougioultzis, S., Keates, A.C., Zhao, D., Peek, R.M., Jr., Shaw, L.M. and Kelly, C.P. (2001) cag+ Helicobacter pylori Induce Transactivation of the Epidermal Growth Factor Receptor in AGS Gastric Epithelial Cells. J. Biol. Chem., 276, 48127-48134.

King, C.R., Borrello, I., Porter, L., Comoglio, P. and Schlessinger, J. (1989) Ligand-independent tyrosine phosphorylation of EGF receptor and the erbB-2/neu proto-oncogene product is induced by hyperosmotic shock. Oncogene, 4, 13-18.

Knebel, A., Rahmsdorf, H., Ullrich, A. and Herrlich, P. (1996) Dephosphorylation of receptor tyrosine kinases as target of regulation by radiation, oxidants or alkylating agents. EMBO J., 15, 5314-5325.

Kyriakis, J.M. and Avruch, J. (2001) Mammalian Mitogen-Activated Protein Kinase Signal Transduction Pathways Activated by Stress and Inflammation. Physiol. Rev., 81, 807-869.

Lemjabbar, H. and Basbaum, C. (2002) Platelet-activating factor receptor and ADAM10 mediate responses to Staphylococcus aureus in epithelial cells. Nat Med, 8, 41-46.

Merlos-Suarez, A., Ruiz-Paz, S., Baselga, J. and Arribas, J. (2001) Metalloprotease-dependent protransforming growth factor-alpha ectodomain shedding in the absence of tumor necrosis factor-alpha-converting enzyme. J Biol Chem, 276, 48510-48517.

Miyazaki, Y., Hiraoka, S., Tsutsui, S., Kitamura, S., Shinomura, Y. and Matsuzawa, Y. (2001) Epidermal growth factor receptor mediates stress-induced expression of its ligands in rat gastric epithelial cells. Gastroenterology, 120, 108-116.

Montero, J.C., Yuste, L., Diaz-Rodriguez, E., Esparis-Ogando, A. and Pandiella, A. (2002) Mitogen-activated protein kinase-dependent and -independent routes control shedding of transmembrane growth factors through multiple secretases. Biochem J, 363, 211-221.

Peschon, J.J., Slack, J.L., Reddy, P., Stocking, K.L., Sunnarborg, S.W., Lee, D.C., Russell, W.E., Castner, B.J., Johnson, R.S., Fitzner, J.N., Boyce, R.W., Nelson, N., Kozlosky, C.J., Wolfson, M.F., Rauch, C.T., Cerretti, D.P., Paxton, R.J., March, C.J. and Black, R.A. (1998) An essential role for ectodomain shedding in mammalian development. Science, 282, 1281-1284.

Prenzel, N., Fischer, O.M., Streit, S., Hart, S. and Ullrich, A. (2001) The epidermal growth factor receptor family as a central element for cellular signal transduction and diversification. Endocr Relat Cancer, 8, 11-31.

Prenzel, N., Zwick, E., Daub, H., Leserer, M., Abraham, R., Wallasch, C. and Ullrich, A. (1999) EGF receptor transactivation by G-protein-coupled receptors requires metalloproteinase cleavage of proHB-EGF. Nature, 402, 884-888.

Rao, G.N. (1996) Hydrogen peroxide induces complex formation of SHC-Grb2-SOS with receptor tyrosine kinase and activates Ras and extracellular signal-regulated protein kinases group of mitogen-activated protein kinases. Oncogene, 13, 713-719.

Rizoli, S.B., Rotstein, O.D. and Kapus, A. (1999) Cell volume-dependent regulation of L-selectin shedding in neutrophils. A role for p38 mitogen-activated protein kinase. J Biol Chem, 274, 22072-22080.

Rosette, C. and Karin, M. (1996) Ultraviolet light and osmotic stress: activation of the JNK cascade through multiple growth factor and cytokine receptors. Science, 274, 1194-1197.

Schägger, H. and von Jargow, G. (1987) Tricine-Sodium Dodecyl Sulfate-Polyacryamide Gel Electrophoresis for the Separation of Proteins in the Range from 1 to 100 kDa. Analytical Biochemistry, 166, 368-379.

Schlessinger, J. (2002) Ligand-induced, receptor-mediated dimerization and activation of EGF receptor. Cell, 110, 669-672.

Sudo, T. and Karin, M. (2000) Assays for JNK and p38 mitogen-activated protein kinases. Methods Enzymol, 322, 388-392.

Sunnarborg, S.W., Hinkle, C.L., Stevenson, M., Russell, W.E., Raska, C.S., Peschon, J.J., Castner, B.J., Gerhart, M.J., Paxton, R.J., Black, R.A. and Lee, D.C. (2002) Tumor necrosis factor-alpha converting enzyme (TACE) regulates epidermal growth factor receptor ligand availability. J Biol Chem, 277, 12838-12845.

Szatrowski, T.P. and Nathan, C.F. (1991) Production of large amounts of hydrogen peroxide by human tumor cells. Cancer Res, 51, 794-798.

Tice, D.A., Biscardi, J.S., Nickles, A.L. and Parsons, S.J. (1999) Mechanism of biological synergy between cellular Src and epidermal growth factor receptor. Proc Natl Acad Sci U S A, 96, 1415-1420.

Umata, T., Hirata, M., Takahashi, T., Ryu, F., Shida, S., Takahashi, Y., Tsuneoka, M., Miura, Y., Masuda, M., Horiguchi, Y. and Mekada, E. (2001) A dual signaling cascade that regulates the ectodomain shedding of heparin-binding epidermal growth factor-like growth factor. J Biol Chem, 276, 30475-30482.

Weskamp, G., Cai, H., Brodie, T.A., Higashyama, S., Manova, K., Ludwig, T. and Blobel, C.P. (2002) Mice lacking the metalloprotease-disintegrin MDC9 (ADAM9) have no evident major abnormalities during development or adult life. Mol Cell Biol, 22, 1537-1544.

Yan, Y., Shirakabe, K. and Werb, Z. (2002) The metalloprotease Kuzbanian (ADAM10) mediates the transactivation of EGF receptor by G protein-coupled receptors. J Cell Biol, 158, 221-226.

The present invention is also described by the following items:
1. Use of an inhibitor of a receptor tyrosine kinase ligand for the manufacture of a medicament for the prevention or treatment of an at least partially therapy-resistant hyperproliferative disorder.
2. The use of item 1 wherein the disorder is cancer.
3. The use of items 1 or 2 wherein the disorder is an at least partially irradiation and/or medicament-resistant cancer.
4. The use of any one of items 1 to 3 wherein the disorder is at least partially resistant against apoptosis-inducing therapy.
5. The use of any one of items 1 to 4 wherein the disorder is at least partially resistant against administration of cytostatic and/or cytotoxic medicaments, particularly apoptosis-inducing medicaments.
6. The use of any one of items 1 to 5 wherein the inhibitor of a receptor tyrosine kinase ligand is co-applied with a further therapeutic procedure and/or medicament.
7. The use of item 6 wherein the medicament is co-applied with an irradiation therapy.
8. The use of items 6 or 7 wherein the medicament is co-applied with a further anti-cancer medicament, particularly with a chemotherapeutic agent or with an anti-tumour antibody.
9. The use of item 8 wherein the further anti-cancer medicament is selected from doxorubicin, a taxane, cis/transplatin or derivatives thereof, 5-fluorouracil, mitomycin D, paclitaxel, etoposide, cyclophosphoamide, docetaxel or other apoptosis-inducing drugs or proteins, in particular antibodies .
10. Use of an inhibitor of a receptor tyrosine kinase ligand for the manufacture of a medicament for increasing the efficacy of therapies against hyperproliferative disorders.
11. Use of an inhibitor of a receptor tyrosine kinase for the manufacture of a medicament for increasing the sensitivity of hyperproliferative disorders against irradiation and/or medicament treatment.
12. Use of an inhibitor of a receptor tyrosine kinase ligand for the manufacture of a medicament for the prevention or treatment of a hyperproliferative disorder which is caused by or associated with stress-induced activation of a receptor tyrosine kinase.
13. The use of item 12, wherein the stress is an oxidative and/or osmotic stress.
14. The use of items 12 or 13, wherein the stress is a p38-mediated stress.
15. The use of items 12 to 14, wherein the disorder is cancer.
16. The use of any one of items 1 to 15 wherein the receptor tyrosine kinase is selected from EGFR and other members of the EGFR family.
17. The use of any one of items 1 to 6, wherein the receptor is EGFR.
18. The method of any one of items 1 to 17 wherein the receptor tyrosine kinase ligand is a ligand binding to the extracellular domain of said receptor tyrosine kinase.
19. The use of any one of items 1 to 18 wherein the receptor tyrosine kinase ligand is selected from HB-EGF, EGF, amphiregulin, betacellulin, epiregulin, TGF-α, neuregulin or heregulin.
20. The use of item 19 wherein the receptor tyrosine kinase ligand is HB-EGF.
21. The use of any one of items 1 to 20 wherein the inhibitor is an inhibitor of a metalloprotease capable of cleaving the receptor tyrosine kinase ligand or an inhibitor of regulatory steps upstream of the metalloprotease.
22. The use of any one of items 1 to 12 wherein the inhibitor is a direct inhibitor of the receptor tyrosine kinase ligand.
23. The use of any one of items 1 to 22 wherein the inhibitor acts on the nucleic acid level.
24. The use of item 23 wherein the inhibitor is a specific transcription inhibitor, particularly selected from anti-sense molecules, ribozymes or RNAi molecules.
25. The use of item 24 wherein the inhibitor is a gene inactivator.
26. The use of any one of items 1 to 22 wherein the inhibitor acts on the protein level.
27. The use of item 26 wherein the inhibitor is a specific protein inhibitor, particularly selected from antibodies or antibody fragments and/or from proteinaceous or low-molecular weight inhibitors.
28. A pharmaceutical composition or kit comprising as active ingredients
   (a) an inhibitor of a receptor tyrosine kinase ligand which is an inhibitor of a metalloprotease capable of cleaving the receptor tyrosine kinase ligand or an inhibitor of regulatory steps upstream of the metalloprotease, and
   (b) a further medicament for the treatment of hyperproliferative disorders.
29. The composition or kit of item 28 which additionally comprises pharmaceutically acceptable carriers, diluents and/or adjuvants.
30. A method of preventing or treating an at least partially therapy-resistant hyperproliferative disorder comprising administrating an inhbitor of a receptor tyrosine kinase ligand to a subject in need thereof.

## Claims

1. Use of an inhibitor of a receptor tyrosine kinase ligand for the manufacture of a medicament for the prevention or treatment of an at least partially therapy-resistant hyperproliferative disorder.

2. The use of claim 1 wherein the disorder is cancer.

3. The use of claims 1 or 2 wherein the disorder is an at least partially irradiation and/or medicament-resistant cancer.

4. The use of any one of claims 1 to 3 wherein the disorder is at least partially resistant against apoptosis-inducing therapy.

5. The use of any one of claims 1 to 4 wherein the disorder is at least partially resistant against administration of cytostatic and/or cytotoxic medicaments, particularly apoptosis-inducing medicaments.

6. The use of any one of claims 1 to 5 wherein the inhibitor of a receptor tyrosine kinase ligand is co-applied with a further therapeutic procedure and/or medicament.

7. The use of claim 6 wherein the medicament is co-applied with an irradiation therapy.

8. The use of claims 6 or 7 wherein the medicament is co-applied with a further anti-cancer medicament, particularly with a chemotherapeutic agent or with an anti-tumour antibody, wherein further anti-cancer medicament is preferably selected from doxorubicin, a taxane, cis/transplatin or derivatives thereof, 5-fluorouracil, mitomycin D, paclitaxel, etoposide, cyclophosphoamide, docetaxel or other apoptosis-inducing drugs or proteins.

9. Use of an inhibitor of a receptor tyrosine kinase ligand for the manufacture of a medicament for increasing the efficacy of therapies against hyperproliferative disorders.

10. Use of an inhibitor of a receptor tyrosine kinase for the manufacture of a medicament for increasing the sensitivity of hyperproliferative disorders against irradiation and/or medicament treatment.

11. Use of an inhibitor of a receptor tyrosine kinase ligand for the manufacture of a medicament for the prevention or treatment of a hyperproliferative disorder which is caused by or associated with stress-induced activation of a receptor tyrosine kinase, wherein the stress is preferably an oxidative and/or osmotic stress, in particular p38-mediated stress.

12. The use of any one of claims 1 to 11 wherein the receptor tyrosine kinase is selected from EGFR and other members of the EGFR family.

13. The method of any one of claims 1 to 12 wherein the receptor tyrosine kinase ligand is a ligand binding to the extracellular domain of said receptor tyrosine kinase.

14. The use of any one of claim 1 to 13 wherein the receptor tyrosine kinase ligand is selected from HB-EGF, EGF, amphiregulin, betacellulin, epiregulin, TGF-α, neuregulin or heregulin.

15. The use of any one of claims 1 to 14 wherein the inhibitor is an inhibitor of a metalloprotease capable of cleaving the receptor tyrosine kinase ligand or an inhibitor of regulatory steps upstream of the metalloprotease.

16. The use of any one of claims 1 to 11 wherein the inhibitor is a direct inhibitor of the receptor tyrosine kinase ligand.

17. The use of any one of claims 1 to 16 wherein the inhibitor acts on the nucleic acid level or on the protein level.

18. A pharmaceutical composition or kit comprising as active ingredients
(a) an inhibitor of a receptor tyrosine kinase ligand which is an inhibitor of a metalloprotease capable of cleaving the receptor tyrosine kinase ligand or an inhibitor of regulatory steps upstream of the metalloprotease, and
(b) a further medicament for the treatment of hyperproliferative disorders, and optionally comprises pharmaceutically acceptable carriers, diluents and/or adjuvants.


SEQUENCE LISTING

<110> Max-Planck-Institut für Biochemie

<120> Inhibition of stress-induced ligand-dependent EGFR
      activation

<130> 31063PWO-GE

<140>
<141>

<150> EP030152094
<151> 2003-07-04

<160> 18

<170> PatentIn Ver. 2.1

<210> 1
<211> 23
<212> RNA
<213> Artificial Sequence

<220>
<221> misc_feature
<222> (22)..(23)
<223> n means a deoxythymidine

<220>
<223> Description of Artificial Sequence:siRNA

<400> 1
aaucacugug gagacauuug cnn                                          23

<210> 2
<211> 23
<212> RNA
<213> Artificial Sequence

<220>
<221> misc_feature
<222> (22)..(23)
<223> n means deoxythymidine

<220>
<223> Description of Artificial Sequence:siRNA

<400> 2
aaacuuccag uguguagaug cnn                                              23

<210> 3
<211> 23
<212> RNA
<213> Artificial Sequence

<220>
<221> misc_feature
<222> (22)..(23)
<223> n means deoxythymidine

<220>
<223> Description of Artificial Sequence:siRNA

<400> 3
aaugaagagg gacacuuccc unn                                              23

<210> 4
<211> 23
<212> RNA
<213> Artificial Sequence

<220>
<221> misc_feature
<222> (22)..(23)
<223> n means deoxythymidine

<220>
<223> Description of Artificial Sequence:siRNA

<400> 4
aaguugccuc cuccuaaacc ann                                              23

<210> 5
<211> 23
<212> RNA
<213> Artificial Sequence

<220>
<221> misc_feature
<222> (22)..(23)
<223> n means deoxythymidine

<220>
<223> Description of Artificial Sequence:siRNA

<400> 5
aaccucgcug caaagaaugu gnn                                              23


<210> 6
<211> 23
<212> RNA
<213> Artificial Sequence

<220>
<221> misc_feature
<222> (11)
<223> y means thymidine

<220>
<221> misc_feature
<222> (22)..(23)
<223> n means deoxythymidine

<220>
<223> Description of Artificial Sequence:siRNA

<400> 6
aagaccuugy aacgacugcu gnn                                              23


<210> 7
<211> 23
<212> RNA
<213> Artificial Sequence

<220>
<221> misc_feature
<222> (22)..(23)
<223> n means deoxythymidine

<220>
<223> Description of Artificial Sequence:siRNA

<400> 7
aacuccaucu guucuccuga cnn                                              23


<210> 8

<211> 23
<212> RNA
<213> Artificial Sequence

<220>
<221> misc_feature
<222> (22)..(23)
<223> nmeans deoxythymidine

<220>
<223> Description of Artificial Sequence:siRNA

<400> 8
aaauugccag cugcgcccgu cnn                                                23

<210> 9
<211> 23
<212> RNA
<213> Artificial Sequence

<220>
<221> misc_feature
<222> (22)..(23)
<223> n means deoxythymidine

<220>
<223> Description of Artificial Sequence:siRNA

<400> 9
aaaguuugcu uggcacaccu unn                                                23

<210> 10
<211> 22
<212> RNA
<213> Artificial Sequence

<220>
<221> misc_feature
<222> (21)..(22)
<223> n means deoxythymidine

<220>
<223> Description of Artificial Sequence:siRNA

<400> 10
aaguaaggcc caggaguguu nn                                                 22

<210> 11
<211> 23
<212> RNA
<213> Artificial Sequence

<220>
<221> misc_feature
<222> (22)..(23)
<223> n means deoxythymidine

<220>
<223> Description of Artificial Sequence:siRNA

<400> 11
aacauagagc cacuuuggag ann                                                23

<210> 12
<211> 21
<212> RNA
<213> Artificial Sequence

<220>
<221> misc_feature
<222> (20)..(21)
<223> n means deoxythymidine

<220>
<223> Description of Artificial Sequence:siRNA

<400> 12
cguacgcgga auacuucgan n                                                  21

<210> 13
<211> 20
<212> DNA
<213> Artificial Sequence

<220>
<223> Description of Artificial Sequence:primer

<400> 13
agtgcagagg actttgagaa                                                    20

<210> 14
<211> 20
<212> DNA
<213> Artificial Sequence

<220>
<223> Description of Artificial Sequence:primer

<400> 14
tgccgttgta gcaataggct                                                    20


<210> 15
<211> 20
<212> DNA
<213> Artificial Sequence

<220>
<223> Description of Artificial Sequence:primer

<400> 15
ttgctcacga agttggacat                                                    20


<210> 16
<211> 20
<212> DNA
<213> Artificial Sequence

<220>
<223> Description of Artificial Sequence:primer

<400> 16
tttcccaggt ttcagtttgc                                                    20


<210> 17
<211> 27
<212> DNA
<213> Artificial Sequence

<220>
<223> Description of Artificial Sequence:primer

<400> 17
ggctggcagt gtcgtcctac cagaggg                                            27

<210> 18
<211> 30
<212> DNA
<213> Artificial Sequence

<220>
<223> Description of Artificial Sequence:primer

<400> 18
ggtgcaccca gctgcagttc agctcagtcc                                        30